# EUROPEAN PATENT SPECIFICATION

(11) **EP 0 295 605 B1**
(45) Date of publication and mention of the grant of the patent: **07.09.1994**
(21) Application number: 88109376.9
(22) Date of filing: 13.06.1988
(51) Int. Cl.: C12P 21/00, C12N 5/00

(54) **Antibody production-stimulating factor derived from human B lymphoblastoid cell and process for producing antibody by using said stimulating factor**
Von menschlichen B-lymphoblastoiden Zellen abstammender Faktor zur Stimulierung der Antikörperproduktion und Verfahren zur Antikörperherstellung unter Verwendung dieses stimulierenden Faktors
Facteur stimulateur de la production d'anticorps produit par les cellules lymphoblastoides B et procédé pour la production d'anticorps avec ce facteur stimulateur

(30) Priority: 12.06.1987 JP 145153/87
(43) Date of publication of application: 21.12.1988
(73) Proprietor: Tosoh Corporation, Shinnanyo-shi, Yamaguchi-ken, 746 (JP)
(72) Inventor: Toyoda, Kazuhisa, Fukuoka-shi Fukuoka (JP); Murakami, Hiroki, Fukuoka-shi Fukuoka (JP); Yamada, Koji, Fukuoka-shi Fukuoka (JP); Omura, Hirohisa, Fukuoka-shi Fukuoka (JP)
(74) Representative: VOSSIUS & PARTNER

(56) References cited:
- FR-A- 2 549 726
- CELL BIOLOGY INTERNATIONAL REPORTS, vol. 10, no. 2, February 1986, pages 77-83,Academic Press Inc., London, GB; H. OHASHI et al.: "HO-323, a human B-lymphoblastoid cell line useful for making human-human hybridomas"

## Description

The present invention relates to a physiologically active substance stimulating the production of an antibody by a hybridoma, and a process for producing an antibody by cultivating an antibody-producing hybridoma by using this active substance.

A monoclonal antibody produced by a hybridoma is a very useful substance for investigations in biology and medicine and has provided new research means for sorting, determination and purification of cell membrane antigens, verification and purification of intercellular active substances and examination of receptors. Moreover, in practical applications, this monoclonal antibody is expected to be used for the diagnosis and remedy of diseases and for refining substances to high purity. In connection with diagnosis and remedy of human diseases, it is thought that,in the case of conventional mouse monoclonal antibodies, the application range will be restricted. Accordingly, the technique of producing human-human hybridomas that produce human monoclonal antibodies has been recently developed. These monoclonal antibodies are produced and secreted by respective hybridomas, and in principle, monoclonal antibodies can be obtained by cultivating hybridomas. But, a technique for preparing high-purity monoclonal antibodies in a large quantity, which is practically satisfactory, has not been established.

As means for obtaining monoclonal antibodies in large quantities, a method has been mainly used in which a hybridoma is cultured in the peritoneal cavity of a mouse. But, culture of a human-human hybridoma in the peritoneal cavity of a mouse is difficult, and moreover, where a mouse hybridoma is cultured in the peritoneal cavity of a mouse, a mixture of a monoclonal antibody produced by the hybridoma and a non-specific mouse antibody produced by the mouse per se is obtained, and a high-purity monoclonal antibody cannot be obtained. Accordingly, the utilization of the culture of hybridomas in the peritoneal cavity of a mouse is extremely restricted.

The production of a large number of hybridoma cells in vitro has been attempted, and in general, a hybridoma shows good growth in a culture medium containing fetal bovine serum (hereinafter referred to as "FCS"), and an antibody is efficiently produced. Nevertheless, serum proteins are present at high concentrations in a culture medium containing FCS, and these proteins have a wide variety of physical and chemical properties and a very broad molecular weight distribution, and accordingly, in serum culture, it is very difficult to refine a monoclonal antibody to high purity. For these reasons, to obtain a large quantity of high-purity monoclonal antibodies, it is necessary to culture a large number of hybridoma cells in a serum-free medium.

In essence, the serum-free medium can provide the environment most suitable for the production of antibodies according to the properties of the hybridoma used, and the serum-free medium is also valuable for separation and purification of antibodies. Only several proteins having known properties are contained in the serum-free medium and the protein content is much lower than in the serum-containing medium, and accordingly, in serum-free culture, the antibody can be separated by simple means and can be easily refined to high purity.

In general, however, the antibody-producing capacity of a hybridoma in a conventional serum-free medium is much lower than in a serum-containing medium. Accordingly, it is necessary to develop a serum-free medium in which a hybridoma can exert an antibody-producing capacity comparable to that in a serum-containing medium, and purification of the produced monoclonal antibodies is not inhibited.

It is an object of the present invention to provide a physiologically active substance stimulating the production of antibody by a hybridoma. Another object of the present invention is to provide a process for producing antibody by cultivating an antibody-producing hybridoma by using this physiologically active substance.

It now has been found that human B lymphoblastoid cells, especially the cell line HO-323 [H. Ohashi et al, Cell Biol. Intern. Reports, 10(2), 77 (1986)], produce a physiologically active substance (hereinafter referred to as "IPSF") stimulating the production of antibody by a hybridoma, and moreover, found that if a hybridoma is cultured in not only a serum-containing medium but also a serum-free medium by addition of this IPSF, the hybridoma secrets a large quantity of monoclonal antibodies.
Figure 1 is a diagram illustrating an example of the relationship between the kinds of the fractions added to the culture medium and the amount of IgM produced by HF10B4;
Fig. 2 is a diagram illustrating another example of the relationship between the kinds of the fractions added to the culture medium and the amount of IgM produced by HF10B4;
Fig. 3 is a diagram illustrating still another example of the relationship between the kinds of the fractions added to the culture medium and the amount of IgM produced by HF10B4; and
Fig. 4 is a diagram illustrating an example of the relationship between the culture period and the cell density.

IPSF can be separated and purified from a supernatant of the human B lymphoblastoid cell, preferably HO-323 culture medium or a lysate of HO-323 cells, and the culture conditions of HO-323 have no relation to the properties of the obtained IPSF. No special conditions are necessary for the culture of HO-323, and HO-323 can grow under ordinary culture conditions appropriately adopted. When IPSF is purified from the supernatant of the cultured medium, cultivation can be performed according to conventional procedures, but, preferably a culture medium having lower protein content is used, because the purification of IPSF can be facilitated if a culture medium contains less proteins.

IPSF having reduced impurity content can be obtained by cultivating HO-323. Cells are inoculated to grow, for example, in a culture medium containing FCS, and then separated and washed. The cells are re-inoculated under conditions suitable for the production of IPSF, for example, in a serum-free culture medium not containing FCS, and cultivated therein.

A commercially available culture medium can be used as the main component of the medium for HO-323. For example, there can be used RPMI-1640 medium, Dulbecco's-modified Eagle's medium (DMEM) and Ham's F12 medium (F12). Preferably, RDF medium (RPMI1640:DMEM:F12 = about 2:1:1 by volume) is used. As the additive to such media, there can be used 1) about 20 to 250 units, preferably about 100 units, of penicillin per ml of the culture medium, 2) about 20 to 250 µg, preferably about 100 µg, of streptomycin per ml of the culture medium, 3) about 5 to 30 mM, preferably 20 mM, of HEPES and 4) about 8 to 20 mM, preferably 14 mM, of NaHCO₃ according to need.

As the culture medium suitable for growing HO-323 cells, there can be used, for example, the above-mentioned RDF medium having incorporated therein about 5 to 10% by volume of FCS based on the total volume of the culture medium. As the medium suitable for the production of IPSF, preferably ITES/RDF medium prepared by adding to RDF medium the following four supplements, that is, 10 µg/ml of insulin, 35 µg/ml of transferrin, 20 µM of ethanolamine and 25 nM of sodium selenite, is used. Where IPSF is produced by using HO-323, a serum component such as FCS need not be particularly added to the culture medium, and IPSF having high purity can be easily prepared by the subsequent refining operation.

In connection with the environment for cultivating HO-323, preferably the temperature is about 35 to 37°C and the cells are cultured in humidity-adjusted air containing about 5 to 10% of carbon dioxide gas. The pH value of the culture medium should be maintained at a slightly alkaline level of about 7.0 to 7.4.

The cell inoculation density suitable for the growth of HO-323 is 5 x 10⁴ to 5 x 10⁵ cells per ml of the culture medium, preferably 2 x 10⁵ cells per ml of the culture medium. If the cells are cultured under these conditions, the cell density is increased to 8 x 10⁵ to 2 x 10⁶ cells per ml of the culture medium in 2 to 4 days. Accordingly, the cells are re-inoculated at the above-mentioned density and the culture is further conducted.

Where IPSF is recovered from the cell lysate, the culture of HO-323 is conducted in the FCS-containing RDF medium or the like until the intended cell number is attained, and the cells are separated by centrifugation or the like, suspended in an appropriate buffer, for example, a phosphate- buffered saline, and sonicated or freeze-and-thawed to prepare the cell lysate. A method may be adopted in which, after the culture of HO-323 in the serum-containing culture medium, HO-323 is then cultured in the serum-free medium and the cell lysate is then prepared. But, even if the culture in the serum-free medium is not conducted, there is no risk of an inclusion of impurities.

Where IPSF is recovered from the cultured medium supernatant, a method may be adopted in which the cells are separated as in the production of the cell lysate and then the supernatant is recovered. But, to obtain IPSF having reduced impurity content, preferably a method is adopted in which separated HO-323 is washed at least once with a synthetic culture medium such as RDF medium, the cells are re-inoculated in a serum-free medium such as ITES/RDF medium at a density of 5 x 10⁵ to 2 x 10⁶ cells per ml of the culture medium, and the culture is conducted. The concentration of IPSF in the culture medium produced by HO-323 changes with the lapse of time, and IPSF is accumulated in the culture medium with the lapse of time. Where the cells are inoculated under the above-mentioned conditions, the cells are generally removed from the culture medium after the cells have been cultured for 3 to 5 days, and the cultured supernatant is recovered.

IPSF can be purified from the above-mentioned cultured supernatant or cell lysate by various methods such as salting-out, ultrafiltration, gel filtration chromatography, ion exchange chromatography, affinity chromatography, hydrophobic chromatography and gel electrophoresis.

The amount of IPSF produced is determined by examining the activity of the IPSF. Namely, the IPSF activity was measured by using a human-human hybridoma HF10B4 (hereinafter referred to as "HF10B4") reacting with IPSF to increase the IgM-producing capacity [H. Murakami et al, In vitro Cell. Develop. Biol., 21, 593 (1985)]. HF10B4 is inoculated in ITES/RDF medium containing 10% of the sample liquid, the IPSF activity of which is to be measured, at a density of 5 x 10⁴ to 2 x 10⁵ cells per ml of the culture medium. The culture is conducted at 37°C in the presence of 5% of CO₂. The amount of IgM in the culture medium supernatant is measured by the enzyme-labeled antibody technique. If the amount of IgM produced after addition of the sample liquid is more than 2 times the amount of IgM produced in the absence of IPSF, IPSF is present in the sample liquid. By adding IPSF obtained according to the present invention to the culture medium of the hybridoma, the amount of antibody produced by the hybridoma is increased. More specifically, where the factor of the present invention is added to a serum-free medium such as ITES/RDF medium, the hybridoma shows an antibody-producing capacity comparable to that obtained in a 10% FCS-containing medium. Moreover, since the amount of IPSF to be added is very little, separation of IPSF from the monoclonal antibody is very easy, and therefore, if the hybridoma is cultured by using a serum-free culture medium containing IPSF, a high-purity monoclonal antibody can be easily purified.

Moreover, although the antibody-producing capacity of the hybridoma is thus improved, the growth rate is not as high as in the serum-containing medium, the culture of the hybridoma per se is easily accomplished, and by cultivating the hybridoma continuously for a long time, a large quantity of monoclonal antibodies can be obtained.

The IPSF of the present invention is produced by the human B lymphoblastoid cell line HO-323 and has the physiological activity of stimulating the production of antibody by a hybridoma. Moreover, by addition of IPSF, the antibody-producing activity of a hybridoma is improved not only in a serum-containing medium but also in a serum-free medium. Furthermore, even though the antibody-producing capacity of the hybridoma is improved in a conventional serum-free medium in which IPSF is supplemented, the hybridoma is not grown at such a high speed as in a serum-containing medium. Accordingly, if mass culture of a hybridoma is carried out in the IPSF-supplemented serum-free medium, a large quantity of monoclonal antibodies can be produced continuously over a long time. Moreover, since the growth rate of the hybridoma is lower than in the serum-containing medium, mass culture of hybridoma per se can be performed very easily. Still further, since IPSF has a very high physiological activity, a sufficient action is obtained only by adding a small amount of IPSF to a serum-free medium, and IPSF can be easily separated from the produced monoclonal antibodies. Accordingly, if a hybridoma is cultured by using the IPSF-containing serum-free medium, high-purity monoclonal antibody can be easily recovered.

The present invention will now be described in detail with reference to the following examples. These examples are only illustrative of the invention and by no means limit the scope of the invention.

### Example 1

To RDF medium containing 100 units/ml of penicillin, 100 µg/ml of streptomycin, 5 mM of HEPES and 14 mM of NaHCO₃ as additives, 10% FCS was added, and HO-323 was suspended in the culture medium at a density of 2 x 10⁵ cells per ml of the culture medium. Then, 10 ml of the cell suspension was poured into each of 25 plastic Petri dishes having a diameter of 10 cm (Falcon #3003 supplied by Nippon Becton-Dickinson). Cells were cultured at 37°C in the presence of 5% of CO₂ for 2 days, and then, the cultured medium was centrifuged, and only cells were recovered. The cells were washed twice with the above-mentioned culture medium and suspended in 200 ml of ITES/RDF medium at a density of 1 x 10⁶ cells per ml of the culture medium, and then 10 ml of the cell suspension was poured into each of 20 Petri dishes having a diameter of 10 cm. Cells were cultured at 37°C for 3 days in the presence of 5% of CO₂ , and then, the cultured medium was centrifuged and the supernatant was obtained.

IPSF was purified from the IPSF-containing supernatant, obtained by the cultivation of HO-323, according to the following method. Namely, the supernatant was filtered by using a filter having a pore size of 0.22 µm (supplied by Millipore), and then, the filtrate was poured into a filter holder (UHP-43 supplied by Toyo Kagaku Sangyo) equipped with an ultrafiltration membrane UF-1000PS, UF-300PS, UF-100PS, UF-50PS or UF-10PS (supplied by Tosoh Corp.) and ultrafiltration was carried out under a nitrogen pressure of about 2 kg/cm². Namely, 100 ml of the filtrate was subjected to ultrafiltration by UF-1000PS, whereby about 10 ml of the concentrate (hereinafter referred to as "UF-1000PS fraction") left on the ultrafiltration membrane and about 90 ml of the filtrate were obtained. Then, the filtrate was filtered by using UF-300PS, and about 10 ml of the concentrate (hereinafter referred to as "UF-300PS fraction") and about 80 ml of the filtrate were similarly obtained. The filtrate was then similarly filtered in succession by using UF-100PS, UF-50PS and UF-10PS and 10 ml each of the supernatant concentrates (hereinafter referred to as "UF-100PS fraction, UF-50PS fraction and UF-10PS fraction") fractionated according to the molecular weight were obtained.

Each of the concentrates was dialyzed against phosphate-buffered saline (PBS) and the internal liquid of the dialysis membrane (hereinafter referred to as "dialysate") was added to human-human hybridoma HF10B4 to examine the effect of stimulating the production of antibody. Namely, HF10B4 was inoculated at a density of 5 x 10⁴ cells per ml of the culture medium into ITES/RDF medium containing 10% of the dialysate and ITES/RDF medium containing 10% of PBS as a comparison. Culture was conducted at 37°C for 3 days in the presence of 5% of CO₂ , and the amount of IgM in the cultured supernatant was measured according to the enzyme-labeled antibody technique.

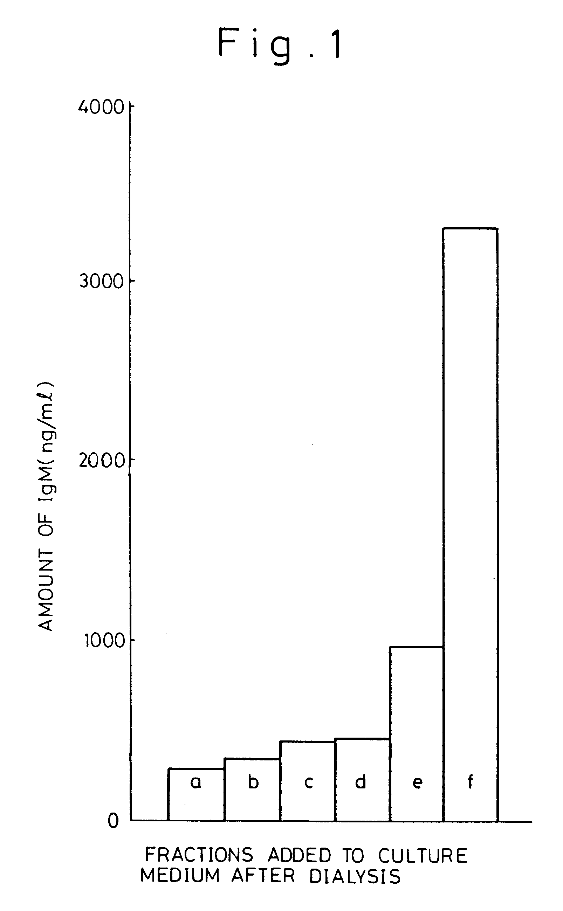

The results are shown in Fig. 1, wherein histograms a, b, c, d, e and f indicate PBS, UF-10PS, UF-50PS, UF-100PS, UF-300PS and UF-1000PS, respectively. As apparent from the results shown in Fig. 1, IPSF could be purified by the ultrafiltration using an ultrafiltration membrane UF-1000PS or UF-300PS.

### Example 2

In the same manner as described in Example 1, HO-323 was cultured in the serum-containing medium and was further cultured in the ITES/RDF medium, and 200 ml of an IPSF-containing cultured supernatant was obtained. Ammonium sulfate was added to the supernatant at a concentration corresponding to 40% of the saturation concentration and the pH value was adjusted to 7.0 by 1N sodium hydroxide. Then, all of the supernatant was passed through a column (30 ml: 16 mm x 15 cm) packed with "Butyl-Toyopearl 650M" (supplied by Tosoh Corp.) and equilibriated with 20 mM Tris-hydrochloric acid (Tris-HCl) buffer (pH 7.0) containing ammonium sulfate at 40% saturation concentration to make IPSF adsorbed. Elution was carried out with 90 ml each of 20 mM Tris-HCl buffers (pH 7.0) containing ammonium sulfate at 30%, 20%, 10% or 0% saturation concentration in sequence. Each of the eluates was poured into a filter holder (UHP-43 supplied by Toyo Kagaku Sangyo) equipped with ultrafiltration membrane UF-30PS (supplied by Tosoh Corp.), and ultrafiltration was carried out under a nitrogen pressure of about 2 kg/cm². By this operation, about 10 ml of the concentrate was obtained on the ultrafiltration membrane in each case.

Each concentrate was dialyzed against PBS, and HF10B4 was inoculated at a density of 1 x 10⁵ cells per ml of the culture medium in ITES/RDF medium to which 10% of the dialysate was added and also in the ITES/RDF medium containing 10% of PBS as a comparison. Culture was conducted at 37°C for 24 hours in the presence of 5% of CO₂ , and the amount of IgM in the cultured supernatant was measured.

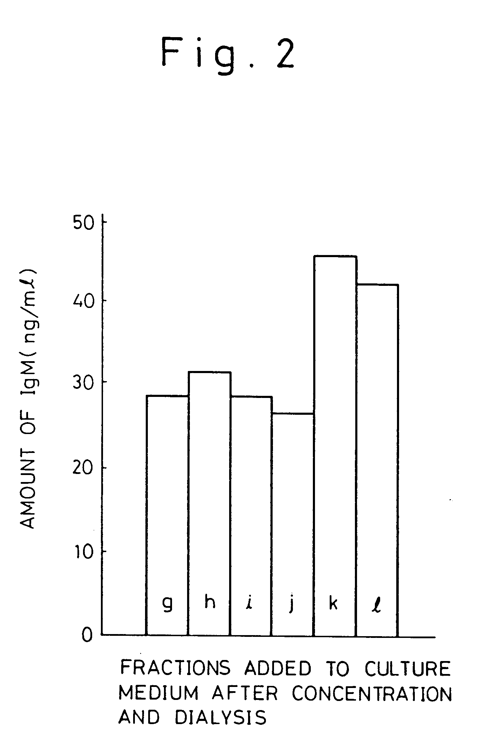

The results are shown in Fig. 2, wherein histograms g, h, i, j, k and l indicate PBS, 40% saturated concentration of ammonium sulfate, 40 - 30% saturated concentration of ammonium sulfate, 30 - 20 % saturated concentration of ammonium sulfate, 20 - 10% saturated concentration of ammonium sulfate, and 10 - 0% saturated concentration of ammonium sulfate, respectively. As apparent from the results shown in Fig. 2, IPSF was eluted in fractions containing ammonium sulfate 20 - 10% saturation concentration and 10 - 0% saturation concentration.

### Example 3

In the same manner as described in Example 1, the supernatant of the medium wherein HO-323 had been cultured was subjected to ultrafiltration using ultrafiltration membranes UF-1000PS and UF-300PS, and concentrates (UF-1000PS fraction and UF-300PS fraction) left on the membranes were recovered.

Each concentrate was dialyzed against PBS, and HF10B4 was inoculated at a density of 2 x 10⁵ cells per ml of the culture medium in ITES/RDF medium containing 10% of the dialysate. Culture was carried out at 37°C for 24 hours in the presence of 5% of CO₂ , and the amount of IgM in the cultured supernatant was measured by the enzyme-labeled antibody technique. Simultaneously, culture was similarly conducted in the ITES/RDF medium containing 10% of FCS instead of the dialysate and the ITES/RDF medium containing 10% of PBS instead of the dialysate, and the amount of produced IgM in each supernatant was measured.

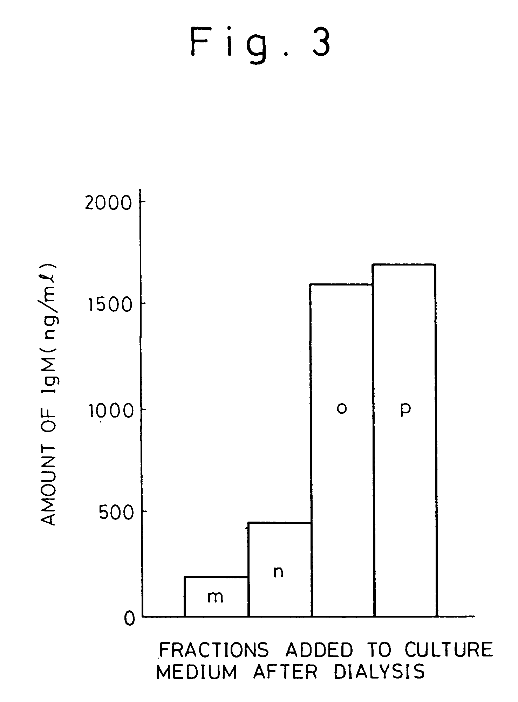

The results are shown in Fig. 3, wherein histograms m, n, o and p indicate PBS, UF-300PS, UF-1000PS and FCS, respectively. It is seen that, when the dialysate of UF-1000PS fraction was added, the amount of the antibody produced by HF10B4 was about 8 times the amount of the antibody produced when PBS was added, and the antibody-producing capacity of HF10B4 was substantially equal to that observed when FCS was added.

### Example 4

In the same manner as described in Example 3, 10% of the dialysate of UF-1000PS fraction of the supernatant of the medium in which HO-323 had been cultured was added to the ITES/RDF medium, and HF10B4 was inoculated at a density of 5 x 10⁴ cells per ml of the culture medium. Culture was carried out at 37°C for 3 days in the presence of 5% of CO₂ , and the cell density in the culture medium was measured with the lapse of time to examine the cell growth rate. Simultaneously, culture was similarly carried out in ITES/RDF medium containing 10% of FCS instead of the dialysate, and also in the ITES/RDF medium containing 10% of PBS instead of the dialysate, and the growth rate was examined.

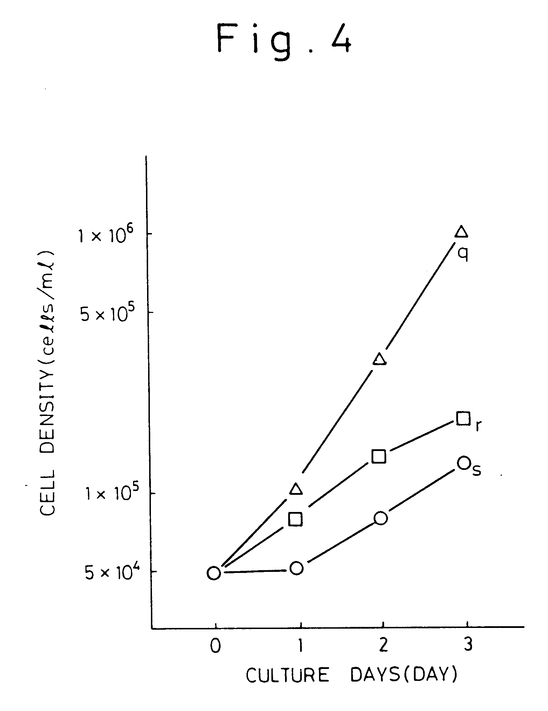

The results are shown in Fig. 4, wherein q, r and s indicate FCS, dialysate and PBS, respectively. It is seen that, when the dialysate was added, the growth rate of HF10B4 was not substantially different from the growth rate obtained when PBS was added, and that the doubling time in the case of an addition of the dialysate was 33.6 hours and the doubling time in the case of an addition of PBS was 36.0 hours. Thus, it was confirmed that the dialysate had no substantial influence on the growth of HF10B4. In contrast, when FCS was added, the doubling time was 15.6 hours, and it was confirmed that FCS dramatically increased the growth rate of cells.

## Claims

1. A physiologically active substance
(i) stimulating the production of antibodies by a hybridoma and
(ii) being obtainable by cultivating human B lymphoblastoid cell line HO-323 and recovering the physiologically active substance from the culture medium and
(iii) being incapable of passing through a UF-300PS ultrafiltration membrane.

2. A physiologically active substance according to claim 1, which is obtainable by cultivating human B lymphoblastoid cell line HO-323 in a medium selected from the group consisting of RPMI 1640 medium, Dulbecco's-modified Eagle's medium (DMEM), Ham's F12 medium and a mixture thereof.

3. A physiologically active substance according to claim 2, wherein the culture medium is a mixture comprised of RPMI 1640 medium, Dulbecco's-modified Eagle's medium and Ham's F12 medium respectively in a proportion by volume of about 2:1:1.

4. A physiologically active substance according to claim 2 or 3, wherein the culture medium contains, per ml of the culture medium, about 20 to 250 units of penicillin, about 20 to 250 µg of streptomycin, about 5 to 30 mM of HEPES and about 8 to 20 mM of NaHCO₃.

5. A physiologically active substance according to any one of claims 2 to 4, wherein the culture medium further contains about 5 to 10% by volume, based on the total volume of the culture medium, of fetal bovine serum.

6. A physiologically active substance according to any one of claims 2 to 5, wherein human B lymphoblastoid cell line HO-323 is cultured at a temperature of about 35 to 37°C in humidity-adjusted air containing about 5 to 10% of carbon dioxide gas and at a pH value of about 7.o to 7.4.

7. A process for the production of antibody, which comprises cultivating an antibody-producing hybridoma in a culture medium which contains a physiologically active substance according to any one of claims 1 to 6.

## Patentansprüche

1. Physiologischer Wirkstoff,
(i) der die Antikörperproduktion eines Hybridoms anregt und
(ii) der durch Züchtung der menschlichen B-lymphoblastoiden Zellinie HO-323 und durch Gewinnung des physiologischen Wirkstoffes aus dem Kulturmedium erhalten werden kann und
(iii) der unfähig ist, die UF-300PS-Ultrafiltrationsmembran zu durchdringen.

2. Physiologischer Wirkstoff nach Anspruch 1, der erhalten werden kann durch Züchtung der menschlichen B-lymphoblastoiden Zellinie HO-323 in einem Medium, ausgewählt aus RPMI 1640-Medium, nach Dulbecco modifiziertem Eagle-Medium (DMEM), Ham-F12-Medium und einem Gemisch davon.

3. Physiologischer Wirkstoff nach Anspruch 2, wobei das Kulturmedium ein Gemisch ist, umfassend RPMI 1640-Medium, nach Dulbecco modifiziertes Eagle-Medium und Ham-F12-Medium in einem Volumenverhältnis von etwa 2:1:1.

4. Physiologischer Wirkstoff nach Anspruch 2 oder 3, wobei das Kulturmedium pro ml Kulturmedium etwa 20 bis 250 Einheiten Penicillin, etwa 20 bis 250 µg Streptomycin, etwa 5 bis 30 mM HEPES und etwa 8 bis 20 mM NaHCO₃ enthält.

5. Physiologischer Wirkstoff nach einem der Ansprüche 2 bis 4, wobei das Kulturmedium weiterhin etwa 5 bis 10 Vol.% fötales Rinderserum bezogen auf das Gesamtvolumen des Kulturmediums enthält.

6. Physiologischer Wirkstoff nach einem der Ansprüche 2 bis 5, wobei die menschliche B-lymphoblastoide Zellinie HO-323 bei einer Temperatur von etwa 35 bis 37°C in Luft mit eingestellter Luftfeuchtigkeit, enthaltend etwa 5 bis 10% Kohlendioxid, und bei einem pH-Wert von etwa 7,0 bis 7,4 gezüchtet wird.

7. Verfahren zur Antikörperherstellung, umfassend die Züchtung eines Antikörper-produzierenden Hybridoms in einem Kulturmedium, das einen physiologischen Wirkstoff nach einem der Ansprüche 1 bis 6 enthält.

## Revendications

1. Substance physiologiquement active qui:
(i) stimule la production d'anticorps par un hybridome et
(ii) peut être obtenue en cultivant la lignée HO-323 de cellules lymphoblastoïdes B humaines et en récupérant la substance physiologiquement active dans le milieu de culture et
(iii) ne peut pas traverser une membrane d'ultrafiltration UF-300PS.

2. Substance physiologiquement active selon la revendication 1 qui peut être obtenue en cultivant la lignée HO-323 de cellules lymphoblastoïdes B humaines dans un milieu choisi parmi le milieu RPMI 1640, le milieu d'Eagle modifié de Dulbecco (DMEM), le milieu F12 de Ham, et un mélange de ces milieux.

3. Substance physiologiquement active selon la revendication 2 dans laquelle le milieu de culture est un mélange comprenant du milieu RPMI 1640, du milieu d'Eagle modifié de Dulbecco et du milieu F12 de Ham respectivement dans un rapport de 2:1:1 en volume.

4. Substance physiologiquement active selon la revendication 2 ou 3 dans laquelle 3 le milieu de culture contient, par ml de milieu de culture, environ 20 à 250 unités de pénicilline, 20 à 250 µg de streptomycine environ, de l'HEPES 5 à 30 mM environ et du NaHCO₃ 8 à 20 mM environ.

5. Substance physiologiquement active selon l'une des revendications 2 à 4 dans laquelle le milieu de culture contient en outre un volume de 5 à 10% de sérum de veau foetal environ, calculé d'après le volume total de milieu de culture.

6. Substance physiologiquement active selon l'une des revendications 2 à 5 dans laquelle la lignée HO-323 de cellules lymphoblastoïdes B humaines est cultivée à une température de 35 à 37°C environ, dans une atmosphère d'un degré d'humidité contrôlé contenant 5 à 10% de gaz carbonique et à un pH de 7,0 à7,4 environ.

7. Procédé de préparation d'un anticorps qui comprend la culture d'un hybridome producteur d'anticorps dans un milieu de culture qui contient une substance physiologiquement active selon l'une des revendications 1 à 6.
